# EUROPEAN PATENT SPECIFICATION

(11) **EP 1 887 975 B1**
(45) Date of publication and mention of the grant of the patent: **01.08.2012**
(21) Application number: 06770292.8
(22) Date of filing: 12.05.2006
(51) Int. Cl.: A61F 2/06

(54) **MEDICAL DEVICE DELIVERY SYSTEMS THAT FACILITATE MEDICAL DEVICE PLACEMENT IN THE PRESENCE OF ULTRASONIC WAVES**
MEDIZINPRODUKT-ABGABESYSTEM ZUR ERLEICHTERUNG DER PLATZIERUNG IN ANWESENHEIT VON ULTRASCHALLWELLEN
SYSTEMES DE DISTRIBUTION DE DISPOSITIFS MEDICAUX FACILITANT LE PLACEMENT DES DISPOSITIFS MEDICAUX EN PRESENCE D'ONDES ULTRASONORES

(30) Priority: 13.05.2005 US 681203 P; 12.05.2006 US 382966
(43) Date of publication of application: 20.02.2008
(73) Proprietor: Cook Medical Technologies LLC, Bloomington, IN 47404 (US)
(72) Inventor: CASE, Brian, C., Lake Villa, Illinois, 60046 (US); Paul, Ram H, Bloomington, Indiana, 47403 (US)
(74) Representative: Garratt, Peter Douglas
(86) International application number: PCT/US2006/018510
(87) International publication number: WO 2006/124647

(56) References cited:
- EP-A- 0 386 936
- US-A- 5 879 305
- US-A1- 2003 028 233
- US-B1- 6 579 221
- US-B1- 6 579 311

## Description

### FIELD

Medical device delivery systems for implanting an intraluminal medical device at a point of treatment in a body vessel are described. Methods of implanting medical devices at a point of treatment and methods of fabricating medical device delivery systems are also described.

### BACKGROUND

Minimally invasive techniques and instruments for placement of intraluminal medical devices have been developed over recent years and are frequently used to deliver an intraluminal medical device to a desired point of treatment and to deploy the intraluminal medical device at the point of treatment. In these techniques, a delivery system is used to carry the intraluminal medical device through a body vessel and to the point of treatment. Once the point of treatment is reached, the intraluminal medical device is deployed from the delivery system for implantation. The delivery system is subsequently withdrawn from the point of treatment and, ultimately, the body vessel. A wide variety of treatment devices that utilize minimally invasive technology have been developed including stents, stent grafts, occlusion devices, infusion catheters, prosthetic valves, and the like.

Some intraluminal medical devices, such as prosthetic valves, include a functional mechanism which is sensitive to positioning in the body vessel. For example, prosthetic venous valves may include a valve orifice that is desirably positioned within a body vessel in a particular orientation. Also, some drug eluting stents may include a drug or other bioactive localized to a particular portion of the medical device, such as a particular circumferential portion. It may be desirable to position the portion containing the drug or other bioactive adjacent a particular portion of the vessel.

Unfortunately, conventional delivery systems do not allow a user to easily position intraluminal medical devices in a particular orientation within the body vessel. Accordingly, there is a need for improved delivery systems for implanting intraluminal medical devices within body vessels.
[0005a] Reference is directed to GB 2 398 245 in which there is provided an implant implantation unit, for inplanting at a determined position in the tubular element with a wall comprising a cavity, is pushed there by a catheter and the unit comprises deformable feelers to, under the control of remote activation elements, change from a stowed form to a deployed functional form, to detect the cavity and position itself there with reference to the position of the cavity. The unit has at least one marker for tagging it, to allow the limit to be imaged by an imaging device. Reference is also directed to EP 1 434 538 in which there is provided a self-expanding stent delivery apparatus having a reinforced sheath for the safe, effective and accurate deployment of self-expanding stents. The sheath is formed from an inner polymeric layer, an outer polymeric layer and a reinforcement layer sandwiched therebetween. The reinforcement layer comprises flat metallic wire to provide the requisite radial and axial strength. In addition, flat wire reduces the profile of the device. Reference is also directed to US 5 879 305 in which there is provided an apparatus and method for introducing an imaging catheter to the coronary vasculature. The distal end of a guiding catheter is shaped so that a mark on the distal end is oriented in a predetermined orientation relative to the coronary vasculature. An imaging catheter is then introduced through the guiding catheter and an image of the mark is produced with the imaging catheter while in the guiding catheter. In this manner, the relative orientation of the produced image and the coronary vasculature is known.

### SUMMARY OF EXEMPLARY EMBODIMENTS

Delivery systems for placing an intraluminal medical device at a point of treatment in a body vessel are described. Delivery systems according to exemplary embodiments facilitate placement of an intraluminal medical device in a particular orientation within the body vessel.

In one example, a delivery system comprises an elongate tubular member having proximal and distal ends and defining a passageway that extends between the ends. The tubular member has first and second wall portions, and the second wall portion is adapted to reflect ultrasonic waves differently than the first wall portion. A dilator that defines a mounting region is disposed within the passageway and an intraluminal medical device is disposed on the mounting region.

The delivery system according to the invention comprises an elongate tubular member having a proximate end and a distal end. The tubular member has a circumferential wall formed of a material and defines a passageway extending between the proximal and distal ends. A dilator that defines a mounting region is disposed within the passageway and an intraluminal medical device is disposed on the mounting region. A first plurality of chambers is formed in the circumferential wall and disposed adjacent a portion of the intraluminal medical device, and a second plurality of chambers is formed in the circumferential wall and disposed adjacent another portion of the medical device. Each chamber of the pluralities of chambers includes a substance that is different from the material of the circumferential wall. The substance in the chambers advantageously has a density that is different than the density of the material of the circumferential wall.

In exemplary embodiments, one or more wall portions of the elongate tubular member that reflect ultrasound waves differently than one or more other wall portions is positioned adjacent a functional mechanism of the intraluminal medical device. In one particular embodiment, such a wall portion is positioned adjacent a structural member of a valve device that at least partially defines a valve orifice. In another particular embodiment, such a wall portion is positioned adjacent a discrete portion of an intraluminal medical device that includes a drug or other bioactive.

Methods of implanting intraluminal medical devices at a point of treatment in a body vessel are also described. One exemplary method comprises the step of providing a medical device delivery system that includes an elongate tubular member that has first and second wall portions that reflect ultrasonic waves differently, a dilator that defines a mounting region disposed within the elongate tubular member, and an intraluminal medical device disposed on the mounting region. In another step, the distal end of the delivery system is advanced through the body vessel until the intraluminal medical device is positioned substantially at a desired point of treatment within the body vessel. In another step, at least a portion of the delivery system is visualized using ultrasonic waves. In another step, the orientation of the intraluminal medical device is verified using the differential reflection of the ultrasonic waves by the first and second wall portions. In an optional step, the delivery system is rotated within the body vessel until a desired orientation of the intraluminal medical device is achieved. In another step, the intraluminal medical device is deployed from the delivery system. In another step, the delivery system is retracted from the body vessel.

Methods of fabricating medical device delivery systems are also described. One exemplary method comprises the step of providing an elongate tubular member that has first and second wall portions that reflect ultrasonic waves differently, a dilator that defines a mounting region, and an intraluminal medical device. In another step, the intraluminal medical device is disposed on the mounting region of the dilator. In another step, the dilator is disposed within a passageway defined by the elongate tubular member. In another step, the circumferential position of at least one of the first and second wall portions is verified relative to one or more portions of the intraluminal medical device. In an optional step, at least one of the elongate tubular member and the dilator is rotated relative to the other until a desired orientation of the intraluminal medical device relative to at least one of the first and second wall portions is achieved.

Additional understanding of the invention can be gained with review of the following detailed description of exemplary embodiments and reference to the appended drawings illustrating the exemplary embodiments.

### BRIEF DESCRIPTION OF THE DRAWINGS

Figure 1 is a perspective view of a delivery system according to a first exemplary embodiment.

Figure 2 is a sectional view of the distal end of the delivery system illustrated in Figure 1.

Figure 3 is a perspective view of the distal end of the delivery system illustrated in Figure 1.

Figure 4 is a sectional view of the delivery system illustrated in Figure 3.

Figure 5 is a sectional view of a delivery system according to an alternate embodiment.

Figure 6 is a sectional view of a delivery system according to another alternate embodiment.

Figure 7 is a sectional view of a delivery system according to another alternate embodiment.

Figure 8 is a schematic sectional view of a body vessel in which the delivery system of Figure 1 is disposed.

### DETAILED DESCRIPTION OF EXEMPLARY EMBODIMENTS

The following detailed description and the appended drawings describe and illustrate various exemplary embodiments. The description and drawings serve to enable one skilled in the art to make and use the invention, and are not intended to limit the scope of the invention, or its protection, in any manner.

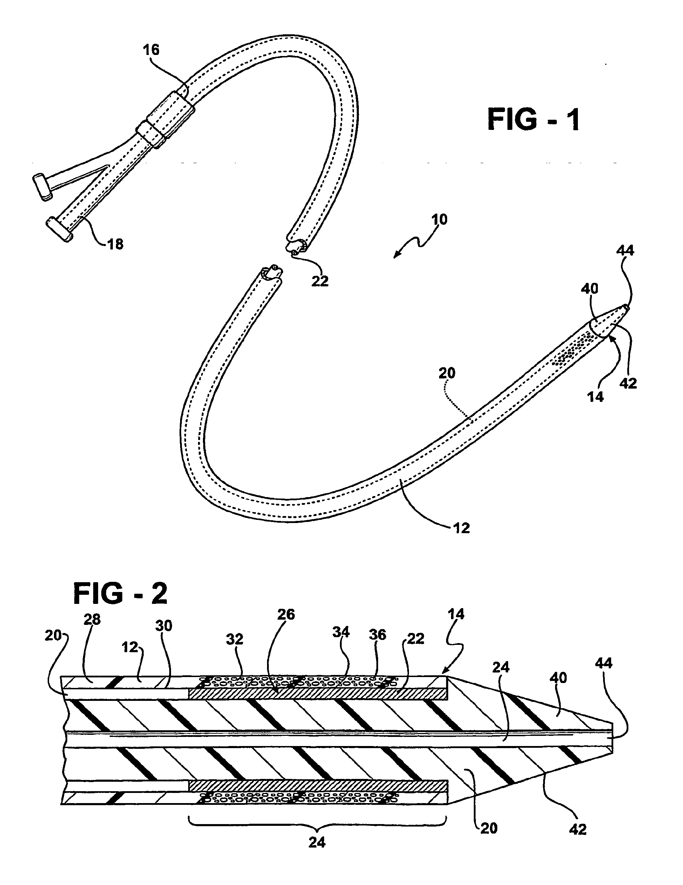

Figures 1 through 4 illustrate a delivery system 10 according to a first exemplary embodiment. The delivery system 10 includes an elongate tubular member 12 having a distal end 14 which is insertable in a body vessel and a proximal end 16 that can be coupled to a connector 18, such as a Touhy-Borst adapter. The elongate tubular member 12 defines a passageway 20 that extends between the proximal 16 and distal 14 ends.

A dilator 22 is disposed in the passageway 20 and defines a mounting region 24. As used herein, the term "dilator" refers to an elongate member capable of being disposed within a lumen of a sheath, such as tubular member 12. An intraluminal medical device 26 is disposed on the mounting region 24.

The elongate tubular member 12 is advantageously formed of a flexible material, such as a plastic or other polymeric material. The elongate tubular member 12 includes a circumferential wall 28 that extends between the proximal 16 and distal 14 ends, and bounds the passageway 20. The circumferential 28 wall includes first 30 and second 32 wall portions.

The second wall portion 32 is adapted to reflect ultrasonic waves differently than the first wall portion 30.

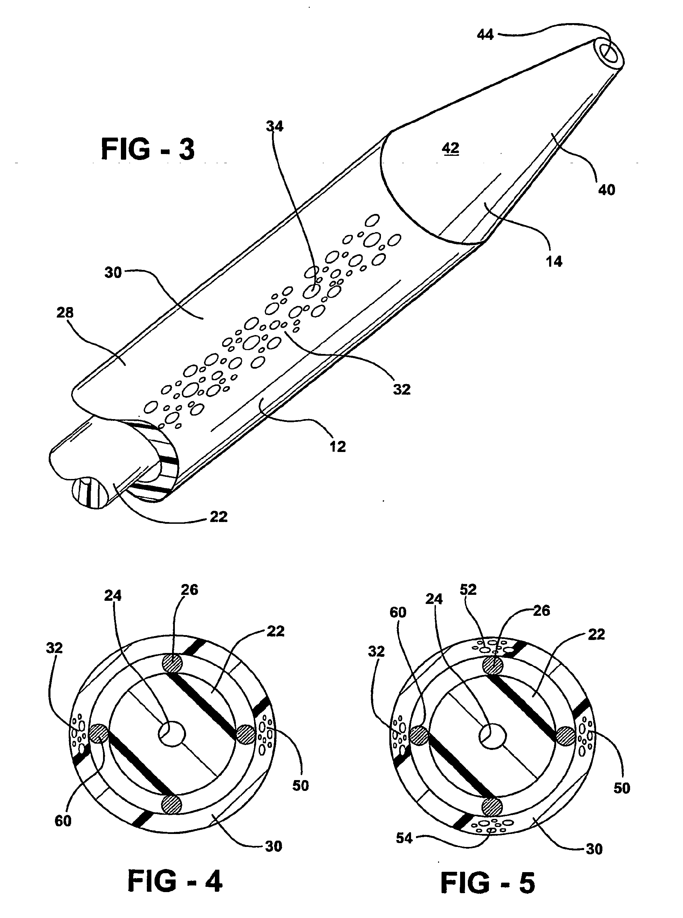

As best illustrated in Figure 3, the second wall portion 32 includes a plurality of chambers 34 formed in the circumferential wall 28. Each chamber 34 includes a substance 36 that is different from the material forming the circumferential wall 28. For plastic elongate tubular members 12, the substance is advantageously a fluid, such as a liquid or gas. It is recognized, though, that other types of substances can be used, including solids and gels. Examples of suitable substances in the chambers 34 include saline and carbon dioxide. Examples of other suitable structural adaptations include the inclusion of a material in the second wall portion that is different than the material of the first wall portion 30. A suitable material can be selected based on relative density compared to the material of the first wall portion 30. If the density of the material is different than the density the material of the first wall portion 30, the desired differential ultrasound reflection will be achieved as the acoustic impedence of a material is directly related to its density. Specific examples of suitable materials for use in the second wall portion include metals and glass particles, which can include a coating. Each of these types of materials is advantageously used with plastic elongate tubular members and can be embedded within the material of the elongate tubular member, disposed on a surface of the elongate tubular member, or both.

A circumferential pattern that includes alternating first 30 and second 32 wall portions, such as that illustrated in Figures 4 and 5, is considered particularly advantageous as it facilitates achievement of a desired orientation of the intraluminal medical device 22 within the body vessel. Furthermore, the second wall portion 32 is to be positioned adjacent a particular portion of the intraluminal medical device 22, such as a functional mechanism, as described in more detail below.

The inclusion of tilled chambers provide the desired differential ultrasound reflection enables the delivery devices according to the invention to be used with ultrasound emitting equipment for visualization purposes, - which offer cost and patient convenience benefits over more costly and less accessible alternative visualization equipment, such as fluoroscopy equipment.

The dilator 22 is disposed within the passageway 20 of the elongate tubular member 12 and provides the mounting region 24 on which the intraluminal medical device 26 is disposed. As best illustrated in Figure 2, the dilator 22 includes a distal end 40 that defines a tapered surface 42 that facilitates placement of the delivery system 10 into a body vessel and its navigation through the body vessel. The dilator 40 defines a wireguide lumen 44. That enables the delivery device to be advanced over a wireguide in conventional fashion. The wireguide lumen 44 can extend along the entire length of the dilator 40, which is useful in over-the-wire applications of the invention, or only along a portion of the entire length of the dilator 40, which is useful in rapid exchange applications. The dilator 40 can be formed of any suitable material. Plastic materials are considered advantageous at least because of their pushability, flexibility, and well-characterized biocompatibility.

The intraluminal medical device 26 can be any suitable intraluminal medical device, including self-expandable devices and devices that require input of additional force to effect expansion, such as balloon expandable intraluminal medical devices. Delivery systems according to the invention are particularly will suited for use in deployment of self-expandable prosthetic devices. Accordingly, a self-expandable prosthetic device can be disposed on the mounting region 24 of the dilator 40. Any suitable type of self-expandable prosthetic device can be used with the delivery systems according to the invention, including self-expandable stents, prosthetic valves that include a self-expandable support frame, such as prosthetic valves for implantation in a vein (prosthetic venous valves), self-expandable filters, distal protection devices, vessel occluders, heart valve devices, and other self-expandable devices. Suitable self-expandable prosthetic devices for use with dlivery systems according to the invention include those described in United States Patent No. 6,200,336 to Pavcnik et al. for a MULTIPLE-SIDED INTRALUMINAL MEDICAL DEVICE; United States Application for Patent Serial No. 101642,372 of Pavcnik et al. for an IMPLANTABLE VASCULAR DEVICE, filed on August 15,2003; and United States Application for Patent Serial No. 10 1828,716 of Case et al. for an ARTIFICIAL VALVE PROSTHESIS WITH IMPROVED FLOW DYNAMICS, filed on April 21, 2004; United States Patent No. 6,096,070 to Ragheb et al. For a COATED IMPLANTABLE MEDICAL DEVICE; and United States Patent No. 6,562,065 to Shanley for an EXPANDABLE MEDICAL DEVICE WITH BENEFICIAL AGENT DELIVERY MECHANISM; which describe suitable intraluminal medical devices for use with delivery systems according to the invention.

The tilled chambers of the second wall portion 32 are disposed adjacent a portion of the intraluminal medical device 26. This positioning allows a user of the delivery system 10 to verify a positioning of the intraluminal medical device 26 within a body vessel prior to deployment. This is particularly useful in deployment procedures of intraluminal medical devices that require or benefit from a particular positioning relative to the vessel itself of another anatomical landmark. For example, some intraluminal medical devices include a functional mechanism that is discretely located on or associated with a portion of the intraluminal medical device. For these types of devices, it may be desirable to orient such functional mechanisms in a particular orientation within the body vessel.

As best illustrated in Figure 4, the delivery system according to the first exemplary embodiment includes second 32 and third 50 wall portions. The third wall portion 50 includes structural adaptations that provide the desired different reflection of ultrasonic waves as compared to reflection by the first wall portion 30. The third wall portion 50 can include the same or different structural adaptations as the second wall portion 32. Any suitable number of wall portions that reflect ultrasonic waves differently than the first wall portion 30 can be used, and the specific number chosen will depend on various considerations, including the structure of the intraluminal medical device 26 and/or the number of functional mechanisms associated with the intraluminal medical device 22. Figure 5 illustrates an alternate embodiment in which second 32, third 50, fourth, 52, and fifth 54 wall portions with different ultrasonic wave reflection properties, as compared to the first wall portion 30, are included.

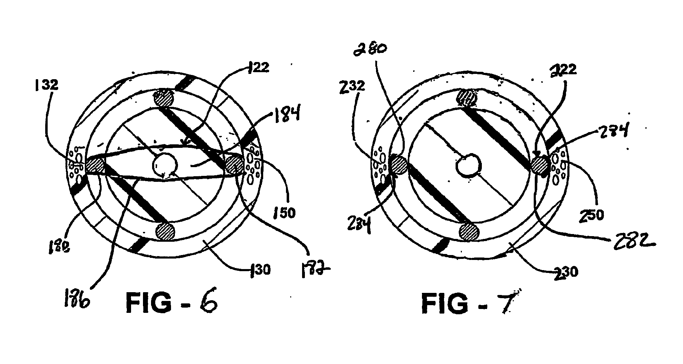

Figure 6 illustrates an alternative embodiment in which the intraluminal medical device comprises a valve device 122 that includes first 180 and second 182 structural members that cooperatively define a valve orifice 184 with an attached graft member 186. Second 132 and third 150 wall portions, which include structural adaptations that cause these portions to reflect ultrasound waves differently than a first wall portion 130, are positioned substantially adjacent the first 180 and second 182 structural members. This allows a user of the delivery system to verify a positioning of the valve orifice 184 of the valve device 122 prior to deployment, which is believed to be advantageous at least because it will allow the orifice to be positioned in an ideal orientation within the body vessel and/or relative to an anatomical landmark of interest, such as a natural valve.

Figure 7 illustrates an alternative embodiment in which the intraluminal medical device comprises an intraluminal support frame 222, such as a stent, that includes first 280 and second 282 structural members that each include an associated coating 284 comprising a drug or other bioactive. Second 232 and third 250 wall portions, which include structural adaptations that cause these portions to reflect ultrasound waves differently than a first wall portion 230, are positioned substantially adjacent the first 280 and second 282 structural members. This allows a user of the delivery system to verify a positioning of the structural members 280, 282 that include the coating 284 prior to deployment, which is believed to be advantageous at least because it will allow the coating 284 to be positioned in an ideal orientation within the body vessel and/or relative to an anatomical landmark of interest, such as a plaque formed on a portion of the vessel wall.

It is noted that structural adaptations that provide the desired differential ultrasound reflection can be included on the dilator of a delivery system according to the invention, such as on the distal end 40 of dilator 22, as best illustrated in Figures 1 through 4. In particular, dimples or other suitable structural adaptations can be formed on the tapered surface 42. In these embodiments, one or more portions of the medical device 26 is advantageously positioned adjacent the dimples or other structural adaptations to provide the desired correlation between the positioning of the medical device 26 and the dilator 22.

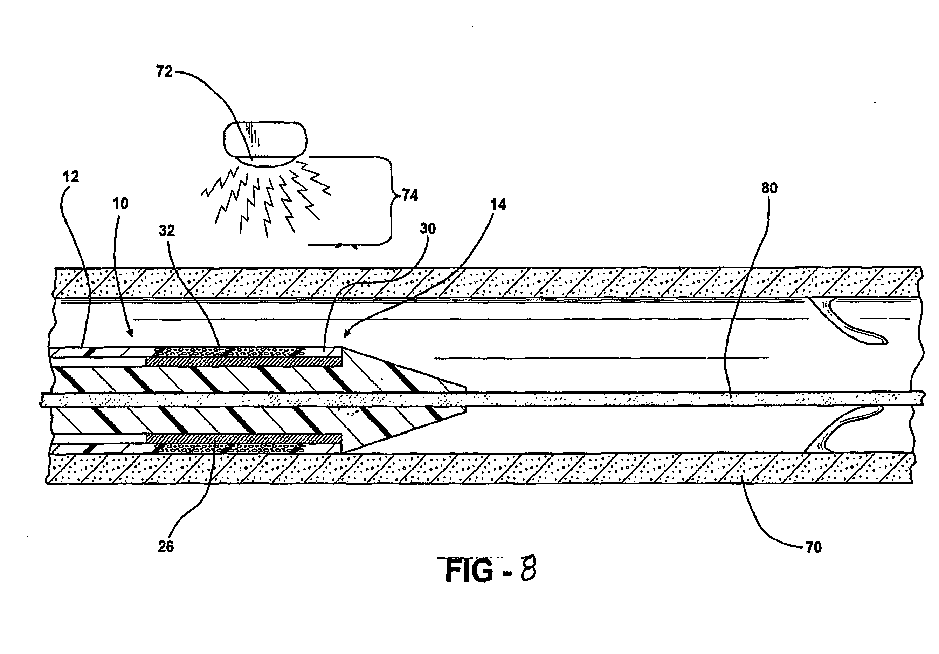

Figure 8 illustrates the distal end 14 of the delivery system 10 within a body vessel 70. An ultrasound emitter 72 is shown directing energy in the form of ultrasonic waves 74 on the portion of the body vessel 70 where the distal end 14 of the tubular member 12 is located. The second wall portion 32 previously described can be differentiated by the ultrasound machine 72 from the first wall portion 30 due to the described differential reflection of the ultrasonic waves 74. A wireguide 80 is disposed in the body vessel 70 to facilitate navigation of the delivery system 10 therethrough.

Methods of implanting intraluminal medical devices at a point of treatment in a body vessel are also provided. One exemplary method comprises the step of providing a medical device delivery system that includes an elongate tubular member that has first and second wall portions that reflect ultrasonic waves differently, a dilator that defines a mounting region disposed within the elongate tubular member, and an intraluminal medical device disposed on the mounting region. In another step, the distal end of the delivery system is advanced through the body vessel until the intraluminal medical device is positioned substantially at a desired point of treatment within the body vessel. In another step, at least a portion of the delivery system is visualized using ultrasonic waves. In another step, the orientation of the intraluminal medical device is verified using the differential reflection of the ultrasonic waves by the first and second wall portions. In an optional step, the delivery system is rotated within the body vessel until a desired orientation of the intraluminal medical device is achieved. In another step, the intraluminal medical device is deployed from the delivery system. In another step, the delivery system is retracted from the body vessel.

Methods of fabricating medical device delivery systems are also provided. One exemplary method comprises the step of providing an elongate tubular member that has first and second wall portions that reflect ultrasonic waves differently, a dilator that defines a mounting region, and an intraluminal medical device. In another step, the intraluminal medical device is disposed on the mounting region of the dilator. In another step, the dilator is disposed within a passageway defined by the elongate tubular member. In another step, the circumferential position of at least one of the first and second wall portions is verified relative to one or more portions of the intraluminal medical device. In an optional step, at least one of the elongate tubular member and the dilator is rotated relative to the other until a desired orientation of the intraluminal medical device relative to at least one of the first and second wall portions is achieved.

It is noted that, while the invention is described and illustrated with reference to medical device delivery systems, it is contemplated and understood that the invention can be equally applied in many different areas of technology, including all areas in which the placement of an article within a passageway in a particular orientation is desirable. For example, the drawing of wires through conduit and the passage of drilling equipment through a passageway could benefit from the invention.

The foregoing detailed description provides exemplary embodiments of the invention. The description and illustration of embodiments is intended only to provide examples of the invention and not to limit the scope of the invention, or its protection, in any manner.

## Claims

1. A medical device delivery system (10) that facilitates medical device placement in the presence of ultrasonic waves, said delivery device comprising: an elongate tubular member (12) having a proximal end (16) a distal end (14), and defining a passageway extending between the proximal and distal ends, the elongate tubular member including first (30, 130,230) and second (32, 132, 232) wall portions, the second wall portion (32, 132,232) adapted to reflect said ultrasonic waves differently than the first wall portion (30, 130, 230).
a dilator (22) disposed within the passageway and defining a mounting region (24); and
an intraluminal medical device (26) disposed on the mounting region; and including a functional mechanism **characterised in that** at least a portion of the functional mechanism is disposed substantially adjacent the second wall portion of the elongate tubular member,
wherein the first wall portion of the elongate tubular member comprises a first material and the second wall portion comprises a plurality of chambers (34), each of the plurality of chambers including a first substance that is different than the material of the first wall portion.

2. The medical device delivery system according to Claim 1, wherein the intraluminal medical device comprises a valve device (122) including first (180) and second (182) structural members cooperatively defining a valve orifice (184) and wherein the second wall portion (132) disposed substantially adjacent at least one of the first (180), and second (182) structural members.

3. The medical device delivery system according to Claim 2, wherein the elongate tubular member includes a third wall portion (150) adapted to reflect said ultrasonic waves differently than the first wall portion (130) and wherein the third wall portion (150) is disposed substantially adjacent at least one of the first and second structural members.

4. The medical device delivery system according to claim 1, wherein the intraluminal medical device comprises a support frame (222), including at least one structural member (280, 282) that includes a coating (284) comprising a bioactive and wherein the second wall portion (232) is disposed substantially adjacent the at least one structural member (280,282).

5. The medical device delivery system according to Claim 4, wherein the at least one structural member comprises a first structural member (280) including a first coating comprising a bioactive and a second structural member (282) including a second coating comprising a second bioactive;
wherein the elongate tubular member includes a third wall portion (250) adapted to reflect said ultrasonic waves differently than the first wall portion; and wherein the second wall portion; and
wherein the second wall portion is disposed substantially adjacent the first structural member and the third wall portion is disposed substantially adjacent the second structural member.

6. The medical device delivery system according to Claim 5 wherein the first and second bioactives are the same.

7. The medical device delivery system according to Claim 5 wherein the first and second bioactives are different.

## Patentansprüche

1. Medizinprodukt-Abgabesystem (10), das die Platzierung eines Medizinprodukts in Anwesenheit von Ultraschallwellen erleichtert, wobei das Abgabeprodukt Folgendes umfasst: ein längliches schlauchförmiges Glied (12), das ein proximales Ende (16), ein distales Ende (14) aufweist und einen Durchgang definiert, der sich zwischen dem proximalen und dem distalen Ende erstreckt, wobei das längliche schlauchförmige Glied einen ersten (30, 130, 230) und einen zweiten (32, 132, 232) Wandabschnitt beinhaltet, wobei der zweite Wandabschnitt (32, 132, 232) geeignet ist, die Ultraschallwellen auf andere Art als der erste Wandabschnitt (30, 130, 230) zu reflektieren; einen Dilatator (22), der innerhalb des Durchgangs angeordnet ist und einen Aufnahmebereich (24) definiert; und
ein intraluminales Medizinprodukt (26), das auf dem Aufnahmebereich angeordnet ist; und einen Funktionsmechanismus beinhaltet, der **dadurch gekennzeichnet ist, dass** mindestens ein Abschnitt des Funktionsmechanismus im Wesentlichen benachbart zum zweiten Wandabschnitt des länglichen schlauchartigen Glieds angeordnet ist; wobei der erste Wandabschnitt des länglichen schlauchförmigen Glieds ein erstes Material umfasst und der zweite Wandabschnitt eine Vielzahl von Kammern (34) umfasst, wobei jede der Vielzahl von Kammern eine erste Substanz beinhaltet, die sich von dem Material des ersten Wandabschnitts unterscheidet.

2. Medizinprodukt-Abgabesystem nach Anspruch 1, wobei das intraluminale Medizinprodukt eine Ventilvorrichtung (122) umfasst, die ein erstes (180) und ein zweites (182) Strukturglied beinhaltet, die zusammenwirkend eine Ventildüse (184) definieren und wobei der zweite Wandabschnitt (132) im Wesentlichen benachbart zu mindestens einem aus dem ersten (180) und zweiten (182) Strukturglied angeordnet ist.

3. Medizinprodukt-Abgabesystem nach Anspruch 2, wobei das längliche schlauchartige Glied einen dritten Wandabschnitt (150) beinhaltet, der geeignet ist, die Ultraschallwellen auf andere Art zu reflektieren als der erste Wandabschnitt (130) und wobei der dritte Wandabschnitt (150) im Wesentlichen benachbart zu mindestens einem aus dem ersten und zweiten Strukturglied angeordnet ist.

4. Medizinprodukt-Abgabesystem nach Anspruch 1, wobei das intraluminale Medizinprodukt einen Trägerrahmen (222) umfasst, der mindestens ein Strukturglied (280, 282) beinhaltet, das eine Beschichtung (284) beinhaltet, die eine bioaktive Substanz umfasst, und wobei der zweite Wandabschnitt (232) im Wesentlichen benachbart zu dem mindestens einen Strukturglied (280, 282) angeordnet ist.

5. Medizinprodukt-Abgabesystem nach Anspruch 4, wobei das mindestens eine Strukturglied ein erstes Strukturglied (280), das eine erste Beschichtung beinhaltet, die eine bioaktive Substanz umfasst, und ein zweites Strukturglied (282) umfasst, das eine zweite Beschichtung beinhaltet, die eine zweite bioaktive Substanz umfasst;
wobei das längliche schlauchförmige Glied einen dritten Wandabschnitt (250) beinhaltet, der geeignet ist, die Ultraschallwellen auf andere Art zu reflektieren als der erste Wandabschnitt; und wobei der zweite Wandabschnitt im Wesentlichen benachbart zum ersten Strukturglied angeordnet ist und der dritte Wandabschnitt im Wesentlichen benachbart zum zweiten Strukturglied angeordnet ist.

6. Medizinprodukt-Abgabesystem nach Anspruch 5, wobei die erste und die zweite bioaktive Substanz gleich sind.

7. Medizinprodukt-Abgabesystem nach Anspruch 5, wobei sich die erste und die zweite bioaktive Substanz voneinander unterscheiden.

## Revendications

1. Système de distribution de dispositifs médicaux (10) qui facilite le placement de dispositifs médicaux en présence d'ondes ultrasonores, ledit dispositif de distribution comprenant: un élément tubulaire allongé (12) qui présente une extrémité proximale (16) et une extrémité distale (14), et qui définit un passage qui s'étend entre les extrémités proximale et distale, l'élément tubulaire allongé comprenant des première (30, 130, 230) et deuxième (32, 132, 232) parties de paroi, la deuxième partie de paroi (32, 132, 232) étant adaptée pour réfléchir lesdites ondes ultrasonores différemment de la première partie de paroi (30, 130, 230),
un dilatateur (22) qui est disposé à l'intérieur du passage et qui définit une région de montage (24); et
un dispositif médical intraluminal (26) qui est disposé sur la région de montage, et comprenant un mécanisme fonctionnel, **caractérisé en ce qu'**au moins une partie du mécanisme fonctionnel est disposée de façon sensiblement adjacente à la deuxième partie de paroi de l'élément tubulaire allongé;
dans lequel la première partie de paroi de l'élément tubulaire allongé comprend un premier matériau, et la deuxième partie de paroi comprend une pluralité de chambres (34), chacune de la pluralité de chambres contenant une première substance qui est différente du matériau de la première partie de paroi.

2. Dispositif médical selon la revendication 1, dans lequel le dispositif médical intraluminal comprend un dispositif de soupape (122) qui comprend des premier (180) et deuxième (182) éléments structurels qui définissent de façon coopérative un orifice de soupape (184), et dans lequel la deuxième partie de paroi (132) est disposée de façon sensiblement adjacente à au moins un des premier (180) et deuxième (182) éléments structurels.

3. Dispositif médical selon la revendication 2, dans lequel l'élément tubulaire allongé comprend une troisième partie de paroi (150) qui est adaptée pour réfléchir lesdites ondes ultrasonores différemment de la première partie de paroi (130), et dans lequel la troisième partie de paroi (150) est disposée de façon sensiblement adjacente à au moins un desdits premier et deuxième éléments structurels.

4. Dispositif médical selon la revendication 1, dans lequel le dispositif médical intraluminal comprend un cadre de support (222) comprenant au moins un élément structurel (280, 282) qui comporte un revêtement (284) comprenant un matériau bioactif, et dans lequel la deuxième partie de paroi (232) est disposée de façon sensiblement adjacente audit au moins un élément structurel (280, 282).

5. Dispositif médical selon la revendication 4, dans lequel ledit au moins un élément structurel comprend un premier élément structurel (280) comprenant un premier revêtement qui comprend un matériau bioactif, et un deuxième élément structurel (282) comprenant un deuxième revêtement qui comprend un deuxième matériau bioactif;
dans lequel l'élément tubulaire allongé comprend une troisième partie de paroi (250) qui est adaptée pour réfléchir lesdites ondes ultrasonores différemment de la première partie de paroi; et
dans lequel la deuxième partie de paroi est disposée de façon sensiblement adjacente au premier élément structurel, et la troisième partie de paroi est disposée de façon sensiblement adjacente au deuxième élément structurel.

6. Dispositif médical selon la revendication 5, dans lequel les premier et deuxième matériaux bioactifs sont identiques.

7. Dispositif médical selon la revendication 5, dans lequel les premier et deuxième matériaux bioactifs sont différents.
